(19) **Europäisches Patentamt**
**European Patent Office**
**Office européen des brevets**

(11) **EP 3 985 677 A1**

(12) **EUROPEAN PATENT APPLICATION**
published in accordance with Art. 153(4) EPC

(43) Date of publication:
**20.04.2022 Bulletin 2022/16**

(21) Application number: **20841170.2**

(22) Date of filing: **10.07.2020**

(51) International Patent Classification (IPC):
***G16B 50/00*** (2019.01)    ***G16B 20/20*** (2019.01)
***G16H 50/70*** (2018.01)

(52) Cooperative Patent Classification (CPC):
**G16B 20/20; G16B 25/10; G16B 40/10;**
**G16B 50/00; G16H 50/20; G16H 50/70**

(86) International application number:
**PCT/KR2020/009095**

(87) International publication number:
**WO 2021/010670 (21.01.2021 Gazette 2021/03)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **12.07.2019 KR 20190084214**

(71) Applicants:
• **Deep Bio Inc.**
**Seoul 08394 (KR)**
• **Gencurix Inc.**
**Seoul 08394 (KR)**

(72) Inventors:
• **KIM, Jee Eun**
**Seoul 08394 (KR)**
• **KANG, Byeongil**
**Seoul 08394 (KR)**
• **LEE, Chang Dae**
**Seoul 08767 (KR)**
• **CHO, Min Ah**
**Seoul 04776 (KR)**

(74) Representative: **Flügel Preissner Schober Seidel**
**Patentanwälte PartG mbB**
**Nymphenburger Straße 20**
**80335 München (DE)**

(54) **DATA PROCESSING METHOD AND SYSTEM USING AUTO-THRESHOLDING**

(57)    Disclosed are a method for automatically calculating a threshold for classifying clusters from a reference data set and processing data by using same, and a system for performing same. According to an aspect of the present invention, provided is a data processing method using auto-thresholding, the data processing method comprising the steps of: receiving, by a data processing system, as an input, a plurality of individual numerical values included in a reference data set having two or more clusters; on the basis of each of the numerical values included in the reference data set received as an input, calculating, by the data processing system, a threshold for classifying a cluster of the reference data set; and classifying, by the data processing system, into different clusters by using the threshold, each of at least one data set to be analyzed, having a plurality of individual numerical values.

FIG. 3

Receiving individual numerical values included in a reference data set — S100

Calculating a threshold for classifying the clusters the reference data set has, based on the individual numerical values included in the reference data set — S110

With respect to at least one or more analysis subject data sets — S120

Classifying the individual numerical values included in the analysis subject data sets using the threshold of the reference data set — S130

**Description**

[Technical Field]

The present invention relates to a data processing method using auto-thresholding and a data processing system for performing the same. In specific, the present invention relates to a data processing method that is capable of automatically calculating a threshold for classifying clusters from a reference data set to perform data processing using the threshold and to a data processing system for performing the same.

[Background Art]

A lot of data are analyzed and utilized in many technology and service fields. For example, a method for analyzing specific medical data to determine whether medicines are applied according to patients or to apply a specialized treatment for an individual patient has been widely used.

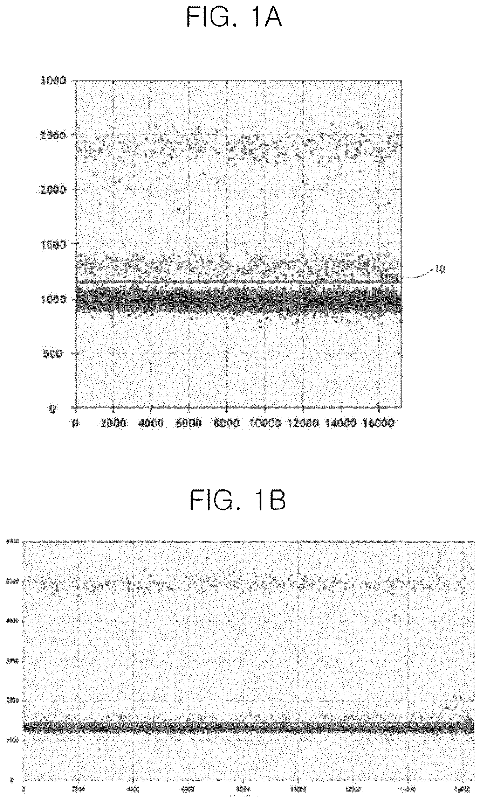

FIG. 1a shows an example of medical data utilized in a given companion diagnostic kit as means for selecting patients proper to a given targeted agent.

FIG. 1a is the medical data obtained by using a test kit (for example, GenesWell™ ddEGFR Mutation Test) indicating existence of mutation on exons 18, 19, 20, and 21 of the EGFR gene as a representative biomarker for a lung cancer, and FIG. 1b shows one experimental data of Droplet Digital™ PCR (ddPCR™) as a method of the GenesWell™ ddEGFR Mutation Test. For example, such medical data are analyzed and used to select patients having effectiveness for a treatment agent before a targeted agent prescription after an operation or to determine whether mutation is expressed or not. However, aspects of the techniques of the present invention are not limitedly applied to the above-mentioned examples, and of course, they may be utilized to analyze various data.

On the graphs as shown in FIGs. 1a and 1b, an x-axis indicates event numbers of the medical data, and a y-axis indicates amplitudes of numerical values of the medical data. Each spot indicates individual medical data. As shown in FIGs. 1a and 1b, the medical data have clustering with at least one cluster (for example, three clusters in FIG. 1a) on a coordinate system.

In this case, there is a need to determine a threshold for classifying clusters in a specific data set, an end point of a specific cluster (for example, at least one individual medical data, that is, at least one data in order from largest values to smallest values of the y-axis) existing on the uppermost position of the lowermost data cluster as a first cluster), or numerical values (values of the y-axis) of the corresponding medical data. However, it is difficult to recognize whether, only with the respective individual medical data, which individual medical data is included in which cluster only through the data numerical values or the coordinates indicated on the coordinate system. In specific, if a plurality of individual medical data exists between the data clusters, the difficulties may become serious.

In conventional practices, as shown in FIGs. 1a and 1b, the individual medical data indicated on the coordinate system are checked with the naked eye to draw the line for indicating a threshold or end point (for example, a top point of a first cluster, that is, a lowermost cluster).

In this case, however, the threshold or end point may be varied according to a person performing the work, thereby undesirably lowering a degree of accuracy.

[Disclosure]

[Technical Problem]

Accordingly, the present invention has been made in view of the above-mentioned problems occurring in the related art, and it is an object of the present invention to provide a data processing method that is capable of automatically calculating a threshold for classifying clusters from a reference data set and performing data processing using the threshold and to a data processing system for performing the same.

It is another object of the present invention to provide a data processing method and system that is capable of automatically searching the end point of a specific data cluster in a data set having two or more data clusters quickly to effectively calculate a threshold.

[Technical Solution]

To accomplish the above-mentioned objects, according to an aspect of the present invention, there is provided a data processing method using auto-thresholding, including the steps of: receiving, as an input, a plurality of individual numerical values included in a reference data set having two or more clusters through a data processing system; calculating a threshold for classifying the clusters the reference data set has through the data processing system, based on the respective numerical values included in the reference data set received; and classifying at least one or more analysis subject data sets having a plurality of individual numerical values into different clusters using the threshold through the data processing system.

According to the present invention, the data processing method may further include the step of calculating a baseline value of the cluster having the smallest average value among the clusters the reference data set has through the data processing system, based on the individual numerical values included in the reference data set received, the step of classifying at least one or more analysis subject data sets having a plurality of individual numerical values into different clusters using the threshold through the data processing system including the steps of: calculating the baseline value of the cluster having the smallest average value among the clusters the reference data set has through the data processing system, based on the individual numerical values included in the reference data set received; calculating a compensation threshold obtained by compensating for the threshold through the data processing system, based on a difference between the baseline value of the reference data set and the baseline value of the analysis subject data sets; and classifying the respective numerical values included in the analysis subject data sets through the data processing system, based on the compensation threshold.

According to the present invention, the respective numerical values included in the reference data set and the at least one or more analysis subject data sets are amplitude values of fluorescent signals measured for droplets obtained by adding a fluorescent dye thereto to detect a specific mutation and then performing a polymerase chain reaction (PCR) to gene sequences corresponding to the specific mutation.

According to the present invention, the step of calculating a threshold for classifying the clusters the reference data set has through the data processing system, based on the respective numerical values included in the reference data set received may include the steps of: producing histogram data having a plurality of bins with a predetermined bin width using the respective numerical values included in the reference data set through the data processing system; performing a noise removing process for allowing the bins having frequencies less than a predetermined noise reference value to have zero frequencies and thus producing histogram data from which noise is removed through the data processing system; searching a first target bin existing on the left end of a first cluster in the reference data set through the data processing system, based on the histogram data from which the noise is removed; searching a second target bin existing on the right end of a second cluster in the reference data set through the data processing system, based on the histogram data from which the noise is removed; and calculating the threshold as any one of the numerical values between the first target bin and the second target bin.

According to the present invention, the step of producing histogram data having a plurality of bins with a predetermined bin width using the respective numerical values included in the reference data set through the data processing system may include the steps of: producing an updated data set from which given top-level numerical values and given bottom-level numerical values are removed from the respective numerical values included in the reference data set; and producing the histogram data using the respective numerical values included in the updated data set.

According to the present invention, the step of calculating a threshold for classifying the clusters the reference data set has through the data processing system, based on the respective numerical values included in the reference data set received may include the steps of: (a) producing histogram data by classifying the range of the numerical values into a plurality of bins having given widths to allow the number of individual data having the respective numerical values of the classified bins to have the frequencies of the respective bins through the data processing system; (b) performing histogram data equalizing through the data processing system; (c) performing differencing for the equalized histogram data through the data processing system; (d) searching a first target bin satisfying a given reference condition and existing on the left end of a first cluster in the reference data set through the data processing system, based on the histogram data with the differencing; (e) searching a second target bin satisfying the given reference condition and existing on the right end of a second cluster in the reference data set through the data processing system, based on the histogram data with the differencing; and (f) calculating the threshold as any one of the numerical values between the first target bin and the second target bin through the data processing system.

According to the present invention, the data processing method may further include the steps of: reducing the bin width by a given value through the data processing system if the first target bin or the second target bin satisfying the given reference condition is not searched; and performing the steps (a) to (e) again using the reduced bin width through the data processing system.

According to the present invention, the step of calculating a threshold for classifying the clusters the reference data set has through the data processing system, based on the respective numerical values included in the reference data set received may include the steps of: (a) producing histogram data by classifying the range of the numerical values into a plurality of bins having given widths to allow the number of individual data having the respective numerical values of the classified bins to have the frequencies of the respective bins through the data processing system; (b) performing histogram data equalizing through the data processing system; (c) searching a first target bin satisfying a given reference condition and existing on the left end of a first cluster in the reference data set through the data processing system, based on the equalized histogram data; and (d) searching a second target bin satisfying the given reference condition and existing on the right end of a second cluster in the reference data set through the data processing system, based on the equalized histogram data.

According to another aspect of the present invention, there is provided a computer program installed in the data processing system to execute the above-mentioned data processing method.

According to yet another aspect of the present invention, there is provided a computer readable recording medium for recording a computer program for executing the above-mentioned data processing method.

According to still another aspect of the present invention, there is provided a data processing system using auto-thresholding, including: an input module for receiving, as an input, a plurality of individual numerical values included in a reference data set having two or more clusters; a threshold calculation module for calculating a threshold for classifying the clusters the reference data set has, based on the respective numerical values included in the reference data set received; and a processing module for classifying at least one or more analysis subject data sets having a plurality of individual numerical values into different clusters using the threshold.

According to the present invention, the data processing system may further include a baseline value calculation module for calculating a baseline value of the cluster having the smallest average value among the clusters the reference data set has, based on the individual numerical values included in the reference data set received, the processing module being adapted to divide the at least one or more analysis subject data sets having the plurality of individual numerical values into different clusters using the threshold by calculating the baseline value of the cluster having the smallest average value among the clusters the analysis subject data sets have, based on the individual numerical values included in the reference data set received, calculating a compensation threshold obtained by compensating for the threshold, based on a difference between the baseline value of the reference data set and the baseline value of the analysis subject data sets, and classifying the respective numerical values included in the analysis subject data sets, based on the compensation threshold.

According to the present invention, the threshold calculation module produces histogram data having a plurality of bins with a predetermined bin width using the respective numerical values included in the reference data set, performs a noise removing process for allowing the bins having frequencies less than a predetermined noise reference value to have zero frequencies to produce histogram data from which noise is removed, searches a first target bin existing on the left end of a first cluster in the reference data set, based on the histogram data from which the noise is removed, searches a second target bin existing on the right end of a second cluster in the reference data set, based on the histogram data from which the noise is removed, and calculates the threshold as any one of the numerical values between the first target bin and the second target bin.

According to the present invention, the threshold calculation module produces an updated data set from which given top-level numerical values and given bottom-level numerical values are removed from the respective numerical values included in the reference data set and produces the histogram data using the respective numerical values included in the updated data set.

According to the present invention, the threshold calculation module produces histogram data by classifying the range of the numerical values into a plurality of bins having given widths to allow the number of individual data having the respective numerical values of the classified bins to have the frequencies of the respective bins, performs histogram data equalizing, performs differencing for the equalized histogram data, searches a first target bin satisfying a given reference condition and existing on the left end of a first cluster in the reference data set, based on the histogram data with the differencing, searches a second target bin satisfying the given reference condition and existing on the right end of a second cluster in the reference data set, based on the histogram data with the differencing, and calculates the threshold as any one of the numerical values between the first target bin and the second target bin.

According to the present invention, the threshold calculation module reduces the bin width by a given value if the first target bin or the second target bin satisfying the given reference condition is not searched, performs the histogram data again using the reduced bin width, and searches the target bin existing on the end of the specific cluster using the histogram data produced again.

According to the present invention, the threshold calculation module produces histogram data by classifying the range of the numerical values into a plurality of bins having given widths to allow the number of individual data having the respective numerical values of the classified bins to have the frequencies of the respective bins, performing histogram data equalizing, searches a first target bin satisfying a given reference condition and existing on the left end of a first cluster in the reference data set, based on the equalized histogram data, searches a second target bin satisfying the given reference condition and existing on the right end of a second cluster in the reference data set, based on the equalized histogram data, and calculates the threshold as any one of the numerical values between the first target bin and the second target bin.

[Effective Advantages of the Invention]

According to the present invention, objective references cluster classification, which are recognized through the reference data set, can be consistently applied to other data sets.

Further, the end point of the specific data cluster can be automatically searched using the numerical values of the individual data quickly, without any separate clustering of the plurality of individual data, so that the threshold as a reference for cluster classification can be effectively and rapidly searched.

In addition, if the data processing method and system according to the present invention is applied to medical data, diagnosis can be more consistently and accurately obtained when compared with manual work in existing methods and systems.

[Description of Drawings]

Now, explanations of drawings are briefly given so as to allow the drawings mentioned in the description to be understood well.

FIGs. 1a and 1b are examples of medical data to which aspects of techniques of the present invention are applied.
FIG. 2 is a block diagram showing a schematic configuration of a data processing system using auto-thresholding according to an embodiment of the present invention.
FIG. 3 is a flowchart showing a data processing method using auto-thresholding according to an embodiment of the present invention.
FIG. 4 is a flowchart showing a process for calculating a threshold through the data processing system according to the present invention.
FIG. 5a shows an example of histogram data and FIGs. 5b and 5c show examples of histogram data from which noise is removed.
FIG. 6a is a flowchart showing steps constituting the step of producing histogram data in the data processing method using auto-thresholding according to the present invention and FIG. 6b shows an example of the histogram data produced using the method as shown in FIG. 6a.
FIG. 7 is a concept view showing an end point searching method of a specific data cluster according to the present invention.
FIG. 8 is a flowchart showing the end point searching method of a specific data cluster according to the present invention.
FIG. 9 is a graph showing an example in which individual data included in a plurality of analysis subject data sets are all classified with respect to the threshold calculated using the data processing method according to the present invention.
FIG. 10 is a flowchart showing a data processing method using auto-thresholding according to another embodiment of the present invention.
FIG. 11 is a graph showing an example in which the threshold is compensated based on a baseline value in the cluster classification for the plurality of analysis subject data sets.
FIG. 12 is a block diagram showing a data processing system using auto-thresholding according to another embodiment of the present invention.

[Best Mode for Invention]

The present invention may be modified in various ways and may have several exemplary embodiments. Specific exemplary embodiments of the present invention are illustrated in the drawings and described in detail in the detailed description. However, this does not limit the invention within specific embodiments and it should be understood that the invention covers all the modifications, equivalents, and replacements within the idea and technical scope of the invention. If it is determined that the detailed explanation on the well known technology related to the present invention makes the scope of the present invention not clear, the explanation will be avoided for the brevity of the description.

Terms, such as the first, and the second, and the like may be used to describe various elements, but the elements should not be restricted by the terms. The terms are used to only distinguish one element from the other element.

Terms used in this application are used to only describe specific exemplary embodiments and are not intended to restrict the present invention. An expression referencing a singular value additionally refers to a corresponding expression of the plural number, unless explicitly limited otherwise by the context.

In this application, terms, such as "comprise", "include", or "have", are intended to designate those characteristics, numbers, steps, operations, elements, or parts which are described in the specification, or any combination of them that exist, and it should be understood that they do not preclude the possibility of the existence or possible addition of one or more additional characteristics, numbers, steps, operations, elements, or parts, or combinations thereof.

When it is said that one element is described as "transmitting" data to the other element, one element may directly transmit data to the other element, but it should be understood that one element may transmit data to the other element through another element. In contrast, when it is said that one element is described as "directly transmitting" data to the other element, it should be understood that one element may transmit data to the other element, without using another element.

The present invention is disclosed with reference to the attached drawings wherein the corresponding parts in the embodiments of the present invention are indicated by corresponding reference numerals.

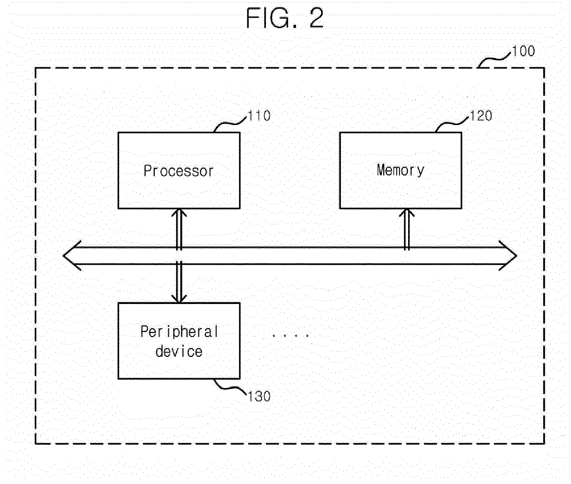

FIG. 2 is a block diagram showing a schematic configuration of a data processing system using auto-thresholding (hereinafter referred to as 'data processing system') according to an embodiment of the present invention.

Referring to FIG. 2, a data processing system 100 according to an aspect of the present invention includes a processor 110 and a memory 120.

The memory 120 stores a computer program (software) for implementing the technical features of the present invention.

The software is executed by the processor 110 to perform a data processing method using auto-thresholding according to the technical features of the present invention.

The data processing system 100 may further include at least one or more peripheral devices 130. The peripheral devices 130 include display devices, speakers, audio/video processing modules, external memories, input/output devices, communication devices, and the like.

According to the present invention, the data processing system 100 is installed on a given server to implement the technical features of the present invention. The server means a data processing device having operation capability with which the technical features of the present invention are implemented, and generally, a data processing device to which a client is accessible through a network and a device capable of performing specific services such as a personal computer, a portable terminal, and the like may be defined as the server, which will be easily understood by a person having ordinary skill in the art. That is, the data processing system 100 may be provided for all kinds of computing systems having data processing capability, such as computers, servers, mobile phones, and the like.

The data processing system 100 is provided as one physical device in FIG. 2, but if necessary, a plurality of physical devices are coupled organically to one another and thus provided as the data processing system 100 according to the technical features of the present invention, which will be easily understood by a person having ordinary skill in the art.

The data processing system 100 receives a given data set. The data set includes a plurality of individual data. The plurality of individual data have respective given values. The values are numerical values. Further, the plurality of individual data may form one data cluster or two or more data clusters.

The clusters are determined by the distribution of the respective individual data in the data set. For example, the individual data, which have distance values equal or less than a given numerical value to thus become close to one another, form one cluster in the data set. Further, the individual data, which have the same attributes as or similar attributes to one another, form one cluster in the entire data set. For example, the entire data set may be classified into a cluster corresponding to mutation expression, a cluster not corresponding to the mutation expression (corresponding to non-expression of mutation), a cluster corresponding to expression of a specific disease, and a cluster not corresponding to the expression of the specific disease (corresponding to non-expression of the specific disease).

The data processing system 100 analyzes a reference data set to calculate a threshold for classifying individual data in another data set to be really analyzed into different clusters and applies the calculated threshold to another data set to be really analyzed to divide the individual data in the corresponding data set into the different clusters.

For example, the data set may be a set of individual data that analyze a sample for detecting expression of a given disease or mutation.

According to an embodiment of the present invention, in specific, the data set may have, as individual data, amplitude values of fluorescent signals measured for droplets obtained by adding a fluorescent dye (for example, FAM probe and/or HEX probe) thereto to detect a specific disease or mutation and then performing a polymerase chain reaction (PCR) to gene sequences (for example, DNA and/or RNA) corresponding to the specific disease or mutation. In this case, the reference data set is a data set corresponding to a sample for positive control, and the analysis subject data set is a data set corresponding to gene sequences extracted from an individual check-up subject.

For example, the data set is an output result from a Droplet Digital™ PCR system. The Droplet Digital™ PCR system divides a 20 microliter (ul) of PCR liquid into about 20,000 droplets, amplifies the droplets, and counts target DNA. According to the amplification of the target DNA of the individual droplets, positive droplets 1 and negative droplets 0 are treated as digital signals and counted, and through Poisson distribution, copies of the target DNA are calculated to finally check result values as the number of copies per ul in a sample. A Droplet Digital™ PCR produces about 20,000 droplets classified through oil films from a PCR liquid containing a sample to be analyzed and a probe (FAM or HEX/VIC probe), performs PCR for the produced droplets, senses the fluorescent signals of the respective droplets through a droplet reader if the PCR is finished, and calculates and analyzes positive droplets, negative droplets, and the number of copies for the target DNA. The analysis result is outputted as a form of a data list (for example, .csv format) having numerical values.

The above-mentioned medical data are given as an example according to the technical features of the present invention, but of course, various data may be used within the scope of the present invention, without being limited thereto.

As mentioned above, the data processing system 100 analyzes the reference data set to calculate a threshold for classifying individual data in another data set to be really analyzed into different clusters and applies the calculated threshold to another data set to be really analyzed to divide the individual data in the corresponding data set into the different clusters. If the data set is a list of numerical values outputted through the Droplet Digital™ PCR, the reference data set is an output result for a positive control sample, and the data set to be really analyzed is an output result for a gene sample extracted from a real check-up subject.

In this case, the data processing system 100 equally applies a threshold calculated from a positive control sample to the results obtained from a plurality of check-up subjects, and accordingly, consistence and objectivity can be ensured when diagnosis for the plurality of check-up subjects is performed.

Further, the data set is a form of a list having numerical values to which event numbers are applied, that is, a form of spreadsheet such as a .csv or .xls file or a form of a database file such as a .db file, or the like.

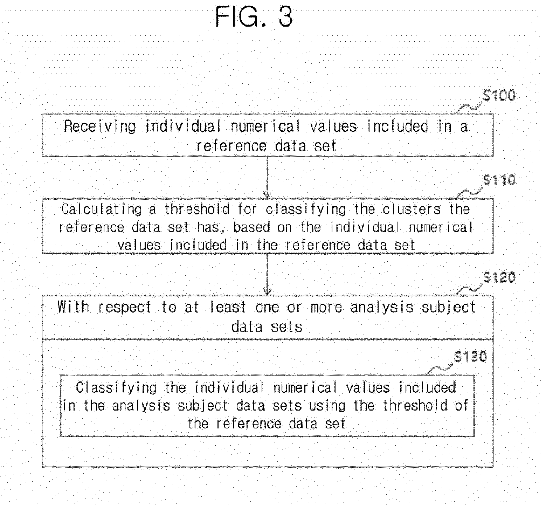

Hereinafter, a process for performing a data processing method using auto-thresholding through the data processing system 100 according to the present invention will be explained in detail below with reference to FIG. 3,.

Referring to FIG. 3, the data processing system 100 receives a plurality of individual numerical values included in a reference data set having two or more clusters (at step S100). The numerical values the individual data have are positive real number values, and a maximum value can be previously determined. According to the present invention, the reference data set is an experimental result for the positive control sample.

After that, the data processing system 100 calculates a threshold for classifying the clusters the reference data set has, based on the received respective numerical values included in the reference data set (at step S110).

According to the present invention, the calculated threshold may be a value for classifying the cluster in which a disease or mutation is expressed and the cluster in which a disease or mutation is not expressed.

There are various methods for calculating the threshold at step S110. So as to calculate the threshold, according to the present invention, the data processing system 100 produces histogram data, while using the received data set, and searches and determines an end point of a specific cluster.

A first axis (for example, x-axis) of the histogram data indicates classes of bins, and a second axis (for example, y-axis) indicates frequencies of respective classes. That is, the histogram data have the range of the numerical values the individual data can have as a domain of the first axis (for example, x-axis) and include, if the first axis is classified into a plurality of bins with a given bin width, information of the respective bins. The information of the respective bins has the range of the first axis value of the corresponding bin (or a bin index indicating the order of the bins and the second axis (for example, y-axis) value of the corresponding bin. The second axis value of the bin is the number of individual data corresponding to the range of the first axis value (that is, the numerical values of the individual data having the range of the bin width).

Further, the end point of the specific cluster may be a left end point or right end point of the corresponding cluster.

The left end point indicates a numerical value of first individual data (or the range of a numerical value just after the numeral value of the first individual data) in the order of high numerical values of the individual data (for example, an upper side in the direction of y-axis in FIG. 1a or 1b) or the range (or the range of a numerical value just after the range) of numerical values some individual data with high numerical values have in the order of high numerical values of the individual data.

The right end point indicates a numerical value of first individual data (or the range of a previous numerical value lower than the numeral value of the first individual data) in the order of low numerical values of the individual data (for example, a lower side in the direction of y-axis in FIG. 1a or 1b) or the range (or the range of a numerical value just after the range) of numerical values predetermined individual data (for example, two to three individual data) with low numerical values have in the order of low numerical values of the individual data.

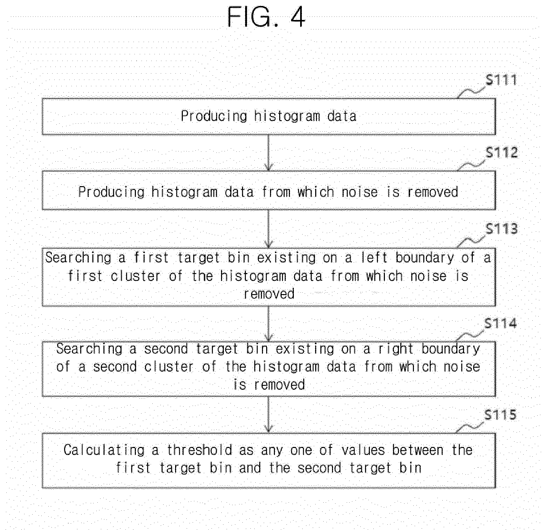

FIG. 4 is a flowchart showing a process for calculating a threshold through the data processing system 100 according to the present invention.

Referring to FIG. 4, the data processing system 100 produces the histogram data having the plurality of bins with a predetermined bin width, while using the respective numerical values included in the reference data set (at step Sill) .

Next, the data processing system 100 performs a noise removing process of allowing the bins having frequencies less than a predetermined noise reference value to have zero frequencies and thus produces the histogram data from which noise is removed (at step S112).

In this case, the noise reference value is a predetermined value obtained through an experiment or other methods.

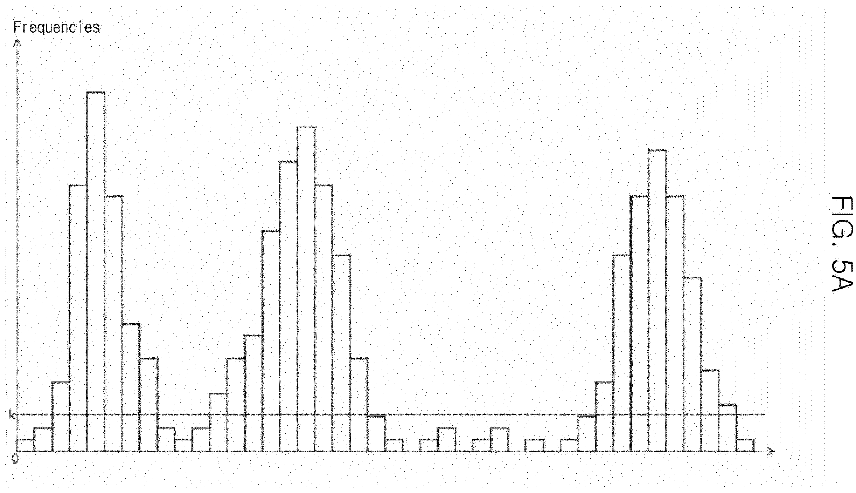

Hereinafter, an example of removing noise from the histogram data will be explained with reference to FIGs. 5a to 5c.

FIG. 5a shows an example of the histogram data produced at step Sill. In specific, the data processing system 100 produces the histogram data as shown in FIG. 5a. In this case, a noise reference value may be determined as k.

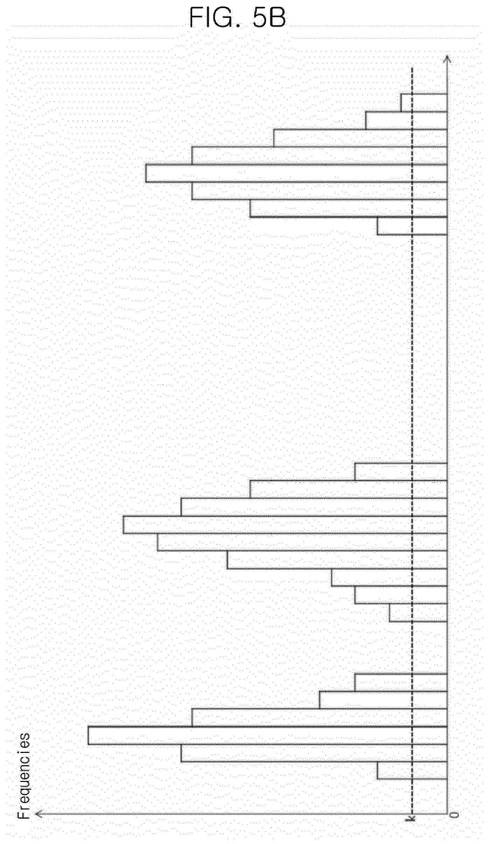

According to the present invention, the noise removing process is a process of setting the bins with the frequencies less than the noise reference value to have zero frequencies. The noise removing process from the histogram data as shown in FIG. 5a is performed, and after that, the histogram data from which the noise is removed is shown in FIG. 5b.

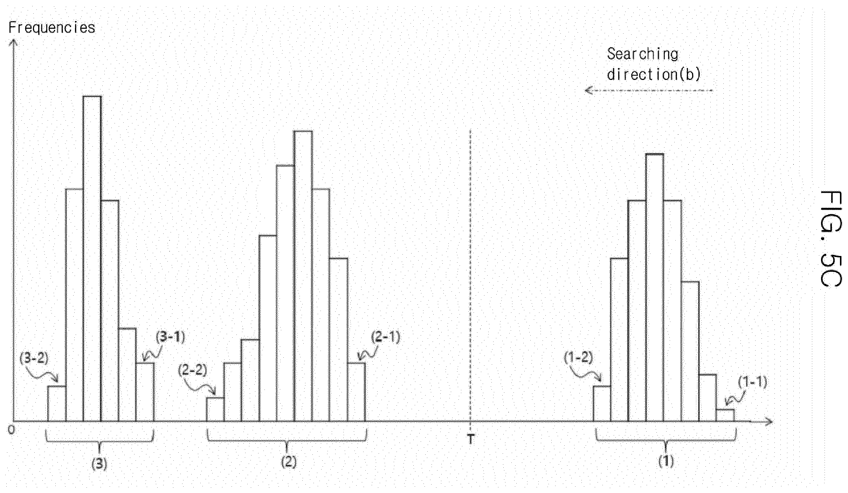

According to the present invention, otherwise, the noise removing process is a process of subtracting the noise reference value from the frequencies of the bins and setting the bins with the frequencies less than zero to have zero frequencies. The noise removing process from the histogram data as shown in FIG. 5a is performed, and after that, the histogram data from which the noise is removed is shown in FIG. 5c.

Referring back to FIG. 4, further, the data processing system 100 after the noise removal from the histogram data searches a first target bin existing on the left end of a first cluster in the reference data set, based on the histogram data from which the noise is removed (at step S113). The first cluster in the reference data set may be the cluster having the highest average numerical value in the clusters the reference data set has.

So as to search the first target bin and a second target bin as will be discussed below, the data processing system 100 searches the respective bins in reverse order from the bin corresponding to the greatest class in the histogram data from which the noise is removed. Referring to FIG. 5c, for example, the data processing system 100 determines the bin 1-1 having the greatest class as the bin existing on the right end of the first cluster and searches the respective bins in reverse order (in a searching direction of b of FIG. 5c), and next, the data processing system 100 determines the bin 1-2 having a frequency greater than zero and whose next bin has a zero frequency as the bin (that is, the first target bin) existing on the left end of the first cluster. Further, the data processing system 100 determines the numerical values existing in the bin 1-2 to the bin 1-1 as the first cluster 1.

Referring back to FIG. 4, the data processing system 100 searches a second target bin existing on the right end of a second cluster in the reference data set, based on the histogram data from which the noise is removed (at step S114). The second cluster in the reference data set may be the cluster having the second highest average numerical value in the reference data set.

Referring to FIG. 5c, for example, after searching the first target bin existing on the left end of the first cluster, the data processing system 100 searches the histogram from which the noise is removed in reverse order and determines the bin 2-1 whose previous bin has a zero frequency and having a frequency greater than zero as a second target bin existing on the right end of the second cluster. Further, the data processing system 100 determines the bin 2-2 having a frequency greater than zero and whose next bin has a zero frequency as the bin existing on the left end of the second cluster, and the data processing system 100 determines the numerical values existing in the bin 2-2 to the bin 2-1 as the second cluster 2.

After searching the bin existing on the left end of the second cluster, next, the data processing system 100 searches the bin 3-1 existing on the right end of a third cluster and the bin 3-2 existing on the left end of the third cluster in the same method as above and determines the third cluster 3.

Referring back to FIG. 4, the data processing system 100 calculates the threshold as one value in values between the first target bin (for example, the first target bin 1-2 of FIG. 5c) and the second target bin (for example, the second target bin 2-1 of FIG. 5c) (at step S115). Referring to FIG. 5c, for example, the data processing system 100 determines an intermediate value T between the maximum value of the second target bin 2-1 and the minimum value of the first target bin 1-2 as the threshold. In addition, the data processing system 100 may determine the threshold through various methods capable of calculating an arbitrary value with which the first cluster 1 and the second cluster 2 are classified.

According to the present invention, the first cluster is the cluster having the highest average numerical value, and the second cluster is the cluster having the second highest average numerical value. However, even in the case where the first cluster is the cluster having the smallest average numerical value and the second cluster is the cluster having the second smallest average numerical value, the technical features of the present invention will be applied. In this case, the data processing system 100 sequentially searches the histogram data from which the noise is removed in order of the bins having the smallest class and determines the left and right ends of the respective clusters, which will be obviously understood to a person having ordinary skill in the art. Further, of course, the data processing system 100 can calculate a threshold with which the second cluster and the third cluster are classified.

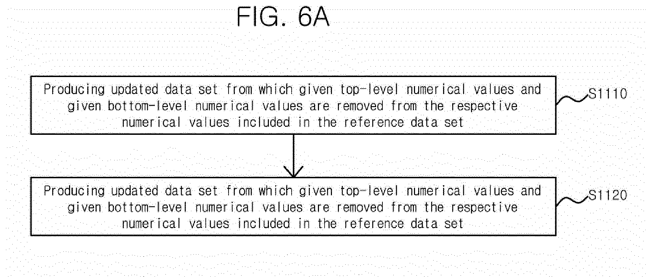

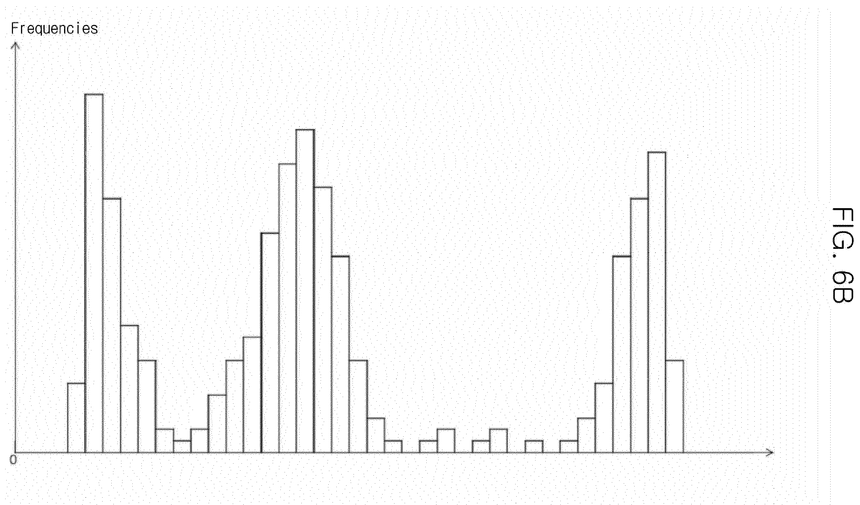

According to another embodiment of the present invention, some of the numerical values, which are unnecessary in calculating the threshold, are removed to thus produce the histogram data, thereby reducing overall operating speed. This will be explained with reference to FIGs. 6a and 6b.

FIG. 6a is a flowchart showing steps constituting the step (Sill of FIG. 4) of producing the histogram data in the data processing method using auto-thresholding according to the present invention.

Referring to FIG. 6a, the data processing system 100 produces an updated data set from which given top-level numerical values and given bottom-level numerical values are removed from the respective numerical values included in the reference data set so as to produce the histogram data (at step S1110). For example, the data processing system 100 removes top 10 percent and bottom 10 percent numerical values from the respective numerical values included in the initial reference data set to thus produce an updated data set.

After that, the data processing system 100 produces the histogram data using the respective numerical values included in the updated data set (at step S1120), and an example of the histogram data produced using the updated data set is shown in FIG. 6b. As mentioned above, FIG. 5a shows an example of the histogram data produced using the initial reference data set, and in the histogram data as shown in FIG. 6b, unlike the histogram data as shown in FIG. 5a, it is checked that the frequencies of the left and right bins are zero.

According to another embodiment of the present invention, the histogram data may not be used, without any change, and in specific, the target bins are searched using histogram data equalization and/or equalized histogram data differencing. According to the present invention, that is, the target bins are searched using the equalized histogram data, and otherwise, they are searched using the equalized histogram data with differencing. In the case of performing the differencing, further, it is easy to more intuitively determine inflection points of the histogram data.

Histogram equalization is a method for converting a series of data so that distributions of the histogram data corresponding to the series of data can appear evenly on the whole area, and for example, the histogram equalization is a technique widely used in a computer vision field for enhancing contrast of image or uniformly adjusting brightness of image. As well known, the histogram equalization is performed by calculating frequencies of respective data to produce a histogram, calculating cumulative frequencies of the respective data, and normalizing the calculated cumulative frequencies.

As widely known, the differencing is a method used in a time series data analysis field to transform time series data having no stationarity so that the data has stationarity. Differencing the series data means that a difference between the series data is calculated, and for example, a differencing method includes a method for calculating a difference between continuous two values (primary differencing), a method for reflecting (adding) white noise ($\varepsilon$) onto the difference between the continuous two values (random walk model), a method for performing re-differencing for the primary difference data (secondary differencing), and a method for performing seasonal differencing to obtain the difference between specific data and previous data from the same season (seasonal differencing).

Further, the histogram equalization and differencing may be performed through a method of applying a mask (or filter) corresponding thereto.

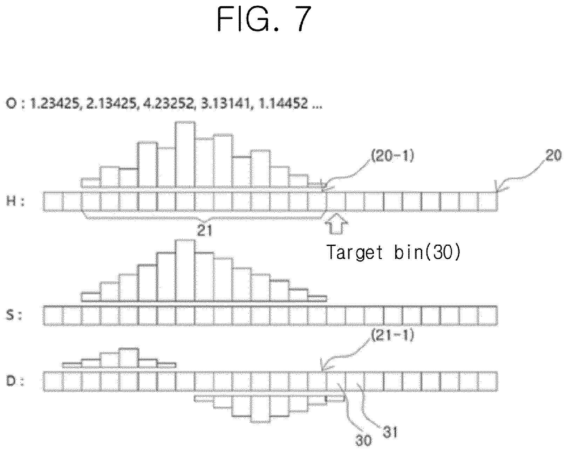

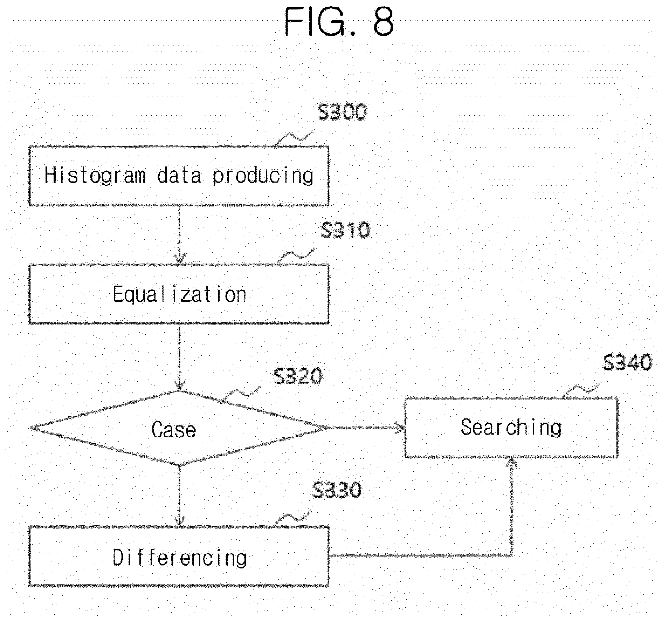

FIG. 7 is a concept view showing an end point searching method of a specific data cluster according to the present invention. As shown in FIG. 7, a symbol ○ indicates original individual data included in a data set, H histogram data, S equalized histogram data, and D histogram data with differencing. Further, FIG. 8 is a flowchart showing the end point searching method of a specific data cluster according to the present invention. For the convenience of the description, an example in which a top point (that is, right end point) of a first data cluster among a plurality of clusters is found in order of low numerical values will be explained below, but of course, the technical features of the present invention are not necessarily applied to the first data cluster or only in the case where the top point of the specific cluster is searched. For example, if the end point of the specific cluster using the histogram data is searched, the end point of an arbitrary data cluster (for example, a second data cluster) may be searched depending on whether which (for example, second) end point is searched in a searching direction (for example, in a direction from the bin having a low numerical value on the first axis to the bin having a high numerical value thereon). Further, the bottom point (that is, the left end point), not the top point of the specific data cluster, may be searched in a searching direction (for example, in a direction from the bin having a high numerical value on the first axis to the bin having a low numerical value thereon). For the convenience of the description, an example in which the top point of the first data cluster among the plurality of clusters is found in order of low numerical values will be explained below, but the spirit and scope of the present invention are not limited thereto.

FIG. 7 shows exemplary histogram data in the case where equalization and/or differencing are performed with a mask having a diameter (that is, the number of parameters) of 3 and parameter values of [-1.0.1], but of course, the diameters or parameter values of the masks for equalization and/or differencing may be freely set.

Referring to FIGs. 7 and 8, the data processing system 100 has the original individual data ○ included in the received data set as positive real numbers (for example, 1.23425, 2.13425, 4.23252, 3.13141, 1.14452, and the like) as shown in FIG. 7. The positive real numbers correspond to the second axis (for example, the y-axis) on the graph as shown in FIG. 1a or 1b.

As mentioned above, the data processing system 100 produces the histogram data H based on the received original individual data ○ (at step S300). The histogram data H are data that are produced by classifying the range of the numerical values of the individual data into a plurality of bins 20 having given widths to allow the number of individual data having the respective numerical values of the classified bins to have the frequencies of the respective bins. If the histogram data is schematically shown, the histogram data H as shown in FIG. 7 is provided.

As shown in FIG. 7, bins 21 having frequencies in the histogram data H are a partial region of the histogram data corresponding to any one data cluster.

Accordingly, an end point of the data cluster, that is, a target bin 30 to be searched by the data processing system 100 is provided as shown in FIG. 7. In FIG. 7, that is, an example in which the bin after the previous bin 21-1 of the target bin 30, not the range of the numerical value (that is, the range of the first axis value of the previous bin 21-1) of the individual data corresponding to the last individual data, i.e., the previous bin 21-1 of the target bin 30, is the target bin, but according to an embodiment of the present invention, of course, the previous bin 21-1 may be the target bin.

The data processing system 100 equalizes the histogram data H, while not directly searching the target bin 30 from the histogram data H.

Accordingly, the data processing system 100 searches the target bin 30 using the equalized histogram data H (at step S340) .

In the case where there is at least one bin (space bin) having a temporary frequency of zero among the series of bins 21 having the frequencies, that is, in the case where there is a range where the individual data do not exist in the range of the number values corresponding to the data cluster to be searched, the equalized histogram data S is used so that it is clearly determined whether the space bin is determined as the target bin or space bin. However, in specific, the space bin in the original histogram H may have a given frequency value, not a zero frequency, according to the left and right frequencies, and therefore, using the equalized histogram S is more effective.

An example obtained by performing equalization for the original histogram data H is the histogram data S as shown in FIG. 7.

Equalizing masks (or filters) and/or differencing masks for equalizing the histogram data are widely known.

According to the present invention, convolution masks are used as the equalizing masks and/or differencing masks, and a given number column x and a convolution mask h is defined by the following mathematical expression.

[Mathematical expression 1]

$$x[n] * h[n] = \sum_{k=-\infty}^{\infty} x[k]h[n-k] = \sum_{l=-\infty}^{\infty} x[n-l]h[l]$$

$$\equiv \sum_{k=-\infty}^{\infty} h[k]x[n-k] = h[n] * x[n]$$

According to an embodiment of the present invention, examples of the equalizing masks and differencing masks are used with [1, 1, 1, 1, 1, 1, 1, 1, 1, 1] and [-1, -1, -1, -1, 0, 1, 1, 1, 1], respectively, and according to another embodiment of the present invention, they are used with [1, 1, 1, 1, 1, 1, 1, 1] and [-1, -1, -1, -1, 0, 1, 1, 1, 1], respectively. However, the equalizing masks and differencing masks may be freely set according to the characteristics of the data set such as the number of individual data included in the data set and clustering of the individual data.

As mentioned above, further, the data processing system 100 searches the target bin 30 using the equalized histogram data S, but in some cases, the data processing system 100 performs differencing for the equalized histogram data S so as to search the target bin 30 more clearly.

In accordance with the characteristics of the data set, in this case, it is previously determined whether the target bin 30 is searched using the equalized histogram data S or using the histogram data D with differencing. The characteristics of the data set are determined on the number of data, densities of data, and the number of data clusters. If the characteristics of the data set are in a given range through experiments repeated previously, that is, in a first case, the target bin 30 may be searched using the equalized histogram data S, and in a second case, the target bin 30 may be searched using the histogram data D with differencing.

According to embodiments of the present invention, of course, any one of the two methods may be randomly selected, and otherwise, after both of the two methods are used to search the target bin 30, the searched results may be compared to each other.

In the case where both of the two methods are used to search the target bin 30, if the respective positions (the values of the first axis) of the searched target bins are the same as each other or within predetermined positions (the values of the first axis), the target bin searched using any one of the two methods may be determined as the final target bin.

Accordingly, if the data processing system 100 determines the first case, based on the original individuation data ○ received (at step S130), the data processing system 100 searches the target bin 30 using the equalized histogram data S (at step S340).

Contrarily, if the data processing system 100 determines the second case, the data processing system 100 performs differencing for the equalized histogram data S (at step S330). Accordingly, the data processing system 100 searches the target bin 30 using the histogram data D with the differencing (at step S340).

An example in which the data processing system 100 searches the target bin 30 using the equalized histogram data S will be explained below.

For example, the data processing system 100 searches the frequencies of the bins of the equalized histogram data S in a given direction (for example, in a direction toward which numerical values are increased).

In this case, if a frequency of a previous bin to a current bin does not have a cut-off value (for example, zero), if a frequency of the current bin has the cut-off value, and if frequencies of bins predetermined in number (for example, one or two or more bins) have the cut-off values, the current bin is determined as the target bin 30.

In specific, if the target bin 30 is the current bin searched currently, the frequency of the previous bin 21-1 is not zero, and as the frequency of the current bin is zero, the frequencies of the previous and next bins (for example, two bins) predetermined in number are zero, so that the current bin can be determined as the target bin 30.

The cut-off value may be zero, but according to the present invention, the cut-off value may be set to have a small value like a number 1. In this case, the end point may be defined by an algorithm that finds only one numerical value of the individual data existing on the end side of the data cluster, and according to embodiments of the present invention, the cut-off value may be freely set.

Contrarily, an example in which the data processing system 100 searches the target bin 30 using the histogram data D with differencing will be explained below.

For example, the data processing system 100 searches the frequencies of the bins of the histogram data D with differencing in a given direction (for example, in a direction toward which numerical values are increased).

In this case, if it is assumed that a current bin is the target bin 30, the current bin may be the target bin 30 to be searched in the case where a frequency of a previous bin 21-1 to the current bin is smaller than a frequency of a bin 31 just after the current bin, the frequency of the previous bin 21-1 is equal to or smaller than zero, and the frequency of the bin 31 is equal to or smaller than zero. That is, a point where the frequency becomes small in a negative value and is then zero can be the target bin 30 to be searched.

When the histogram data is produced, as mentioned above, the target bin 30 may not be searched depending on the widths of bins. For example, if the widths of bins are too big, a plurality of individual data may exist in relative high density between the data cluster to be searched and next data cluster, so that the bin having a cut-off value may not exist. Contrarily, if the widths of bins are too small, a plurality of bins having the cut-off value exist in one data cluster, and otherwise, the number of bins is increased to cause the searching time to be extended. Accordingly, it is necessary to previously determine the appropriate widths of bins through repeated experiments.

If it is hard to previously determine the appropriate widths of bins, searching is performed using a given default bin width value, and if the target bin is not searched (that is, if the bin widths are big so that there is no bin having a zero frequency between the end bin of the target data cluster to be searched and the target data cluster side end point of the data cluster adjacent to the target data cluster), the bin widths are reduced sequentially by a predetermined unit value. As a result, the histogram data may be produced again using the bin widths reduced. After that, the target bin searching (using the equalized histogram data or the histogram data with differencing) as mentioned above may be performed using the produced histogram data.

The data processing system 100 determines the left and right end points of the respective clusters using the above-mentioned methods as shown in FIGs. 7 and 8 and calculates the threshold for classifying the respective clusters using the left and right end points.

Referring back to FIG. 3, after the data processing system 100 calculates the threshold through the above-mentioned methods, the data processing system 100 divides at least one or more analysis subject data sets into different clusters using the threshold (at steps S120 and S130).

The at least one analysis subject data sets include a plurality of individual data, and the respective individual data may have numerical values.

The analysis subject data sets are data produced through a test or experiment performed in the same manner as the reference data set. If the reference data set is a data set measured from a positive control sample related to expression of a specific disease or mutation, the at least one analysis subject data sets are data sets measured from samples having biometric information (for example, gene information) extracted from analysis subjects.

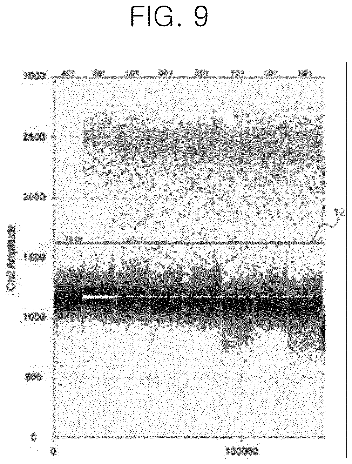

FIG. 9 is a graph showing an example in which individual data included in a plurality of analysis subject data sets are all classified with respect to the threshold calculated using the above-mentioned method.

As shown in FIG. 9, after a threshold 12 as a reference for classification is calculated, the data processing system 100 divides a plurality of analysis subject data sets (A01, B01, ..., and H01) into data clusters (that is, having values greater than the threshold 12) in which the expression of disease or mutation appears and data clusters (that is, having values less than the threshold 12) in which the expression of disease or mutation does not appear.

Further, the plurality of analysis subject data sets may be entirely shifted in numerical values due to errors generated from experimental equipment (for example, Droplet Digital™ PCR system) itself. That is, no problems may occur in one analysis subject data set, but numerical values may be entirely increased or decreased in the relation between the plurality of analysis subject data sets.

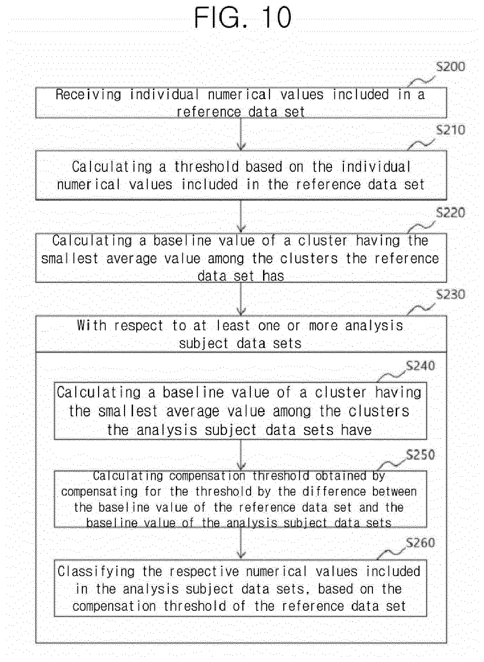

To do this, the data processing system 100 may further include a process of compensating for the whole numerical values, based on a baseline of each analysis subject data set. A specific example of performing such a process in the data processing method using auto-thresholding according to the present invention is shown in FIG. 10.

Referring to FIG. 10, the data processing system 100 receives a plurality of individual numerical values included in a reference data set (at step S200) and calculates a threshold for classifying clusters the reference data set has, based on the individual numerical values included in the reference data set received (at step S210).

Further, the data processing system 100 calculates a baseline value of the cluster having the smallest average value among the clusters the reference data set has, based on the individual numerical values included in the reference data set received (at step S220).

According to an embodiment of the present invention, the data processing system 100 calculates the baseline value through the end point searching method of the specific cluster as mentioned above. For example, the data processing system 100 searches top and bottom points of a specific group (for example, the lowermost group) and calculates an intermediate value, average value, or weight center value between both points as the baseline value.

Further, the data processing system 100 performs steps S240 to S260 for at least one or more analysis subject data sets (at step S230).

The data processing system 100 calculates the baseline value of the cluster having the smallest average value among the clusters the analysis subject data sets have, based on the individual numerical values included in the analysis subject data sets (at step S240).

Further, the data processing system 100 calculates a compensation threshold obtained by compensating for the threshold, based on a difference between the baseline value of the reference data set and the baseline value of the analysis subject data sets.

For example, the data processing system 100 calculates the compensation threshold obtained by compensating for the threshold by the difference between the baseline value of the reference data set and the baseline value of the analysis subject data sets (at step S250). The respective numerical values included in the analysis subject data sets are classified, based on the compensation threshold (at step S260). According to the present invention, further, the data processing system 100 calculates the compensation threshold obtained by compensating for the threshold by the difference between the baseline value of the reference data set and the baseline value of the analysis subject data sets (at step S250) only in the case where the difference between the baseline value of the reference data set and the baseline value of the analysis subject data sets is greater than a given value, and next, the respective numerical values included in the analysis subject data sets are classified, based on the compensation threshold (at step S260).

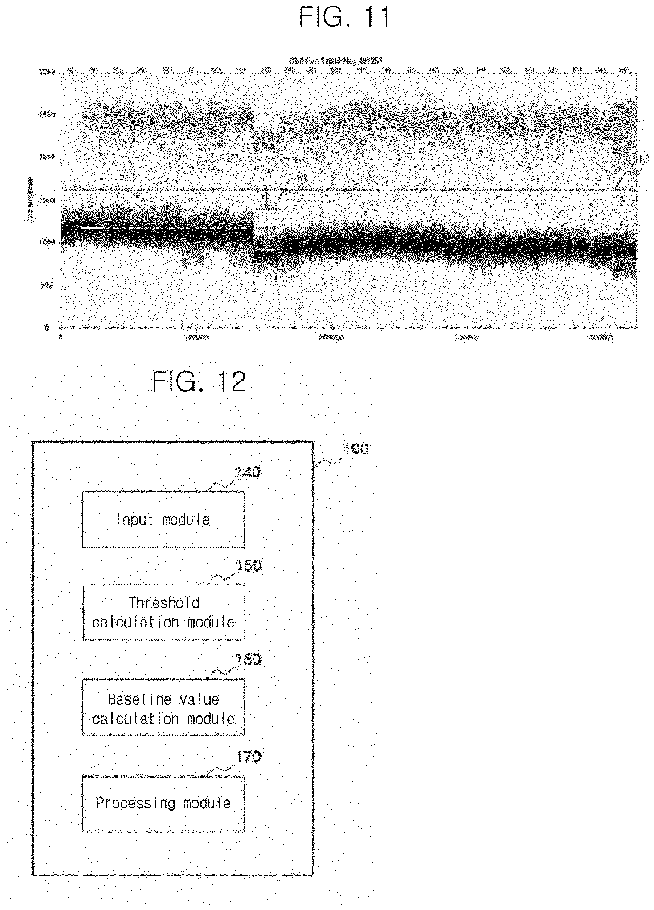

FIG. 11 is a graph showing an example in which the threshold is compensated, based on the baseline value in the cluster classification for the plurality of analysis subject data sets. FIG. 11 shows cluster classification results for the respective data sets (A01, B01, C01, ..., and H09).

Referring to FIG. 11, the data processing system 100 divides the clusters for the remaining data sets except the data set A05, based on a given threshold 13, but divides the clusters of the data set A05 having a different baseline value from the baseline value of the reference data set, based on a threshold compensated by a difference between both baseline values.

FIG. 12 is a block diagram showing a logical configuration of a data processing system 100 according to another embodiment of the present invention.

Referring to FIG. 12, the data processing system 100 includes an input module 140, a threshold calculation module 150, and a processing module 170. According to the present invention, some of the above-mentioned components may not be necessarily required, and the data processing system 100 according to the present invention may further include a larger number of components. For example, the data processing system 100 may further include a baseline value calculation module 160 and/or a control module (not shown) for controlling the components (for example, the input module 140, the threshold calculation module 150, the baseline value calculation module 160, and the processing module 170) or the functions or resources thereof.

The data processing system 100 means a logical configuration having hardware resource and/or software required to implement the technical features of the present invention and does not mean one physical component or device. That is, the data processing system 100 means a logical combination of hardware and/or software required to implement the technical features of the present invention, and so as to implement the technical features of the present invention, if necessary, the data processing system 100 may be provided as a set of logical components installed on separated devices from each other to execute respective functions. Further, the data processing system 100 may be provided as a set of components operating separately by function or role to implement the technical features of the present invention. For example, the input module 140, the threshold calculation module 150, the baseline value calculation module 160, and the processing module 170 may be located on different physical devices from one another, and otherwise, they may be located on the same physical device as one another. According to the present invention, further, combinations of software and/or hardware constituting the input module 140, the threshold calculation module 150, the baseline value calculation module 160, and the processing module 170, respectively may be located on different physical devices from one another, and the components located on the different physical devices may be organically coupled to constitute the respective modules.

Further, a term 'module' used in the description means a functional and structural combination of hardware for implementing the technical features of the present invention and software for driving the hardware. For example, the module means a given code and a logical unit of a hardware resource through which the given code is implemented, and the module does not necessarily mean a code connected physically or one kind of hardware, which is easily understood by a person having ordinary skill in the art.

Referring to FIG. 12, the input module 140 receives the plurality of individual numerical values included in the data set (for example, data set corresponding to positive control) having two or more clusters. The input module 140 receives the reference data set and/or at least one or more analysis subject data sets.

The threshold calculation module 150 calculates a threshold for classifying the clusters the reference data set has, based on the respective numerical values included in the reference data set received. The methods for calculating the threshold through the threshold calculation module 150 have been already described above.

The processing module 170 divides each analysis subject data set having a plurality of individual numerical values into different clusters using the threshold.

According to the present invention, the data processing system 100 further includes the baseline value calculation module 160 for calculating a baseline value of the cluster having the smallest average value among the clusters the reference data set has, based on the individual numerical values included in the reference data set received, and so as to divide the analysis subject data sets having the plurality of individual numerical values into different clusters using the threshold, in this case, the processing module 170 calculates a baseline value of the cluster having the smallest average value among the clusters the analysis subject data sets have, based on the individual numerical values included in the analysis subject data sets, and then calculates a compensation threshold obtained by compensating for the threshold by a difference between the baseline value of the reference data set and the baseline value of the analysis subject data sets.

According to the present invention, further, the threshold calculation module 150 searches the end point of a specific cluster to calculate the threshold.

According to embodiments of the present invention, further, the data processing system 100 may include a processor and a memory for recording a program executed by the processor. The processor may include a single core CPU or multi-core CPU. The memory may include a high speed random access memory, one or more magnetic disc storage devices, a flash memory, or a non-volatile memory such as a non-volatile solid state memory. Access to the memory through the processor and other components is controlled by means of a memory controller.

Further, the data processing method using auto-thresholding according to the present invention may be implemented in the form of a program instruction that can be performed through computers, and may be recorded in a computer readable recording medium. According to the present invention, further, a control program and a subject program may be recorded in a computer readable recording medium. The computer readable recording medium may include all kinds of recording devices in which data readable by a computer system are recorded.

The program instruction recorded in the recording medium is specially designed and constructed for the present invention, but may be well known to and may be used by those skilled in the art of computer software.

The computer readable recording medium may include a magnetic medium such as a hard disc, a floppy disc, and a magnetic tape, an optical recording medium such as a compact disc read only memory (CD-ROM) and a digital versatile disc (DVD), a magneto-optical medium such as a floptical disk, and a hardware device specifically configured to store and execute program instructions, such as a read only memory (ROM), a random access memory (RAM), and a flash memory. Further, the computer readable recording medium is distributed over network-coupled computer systems so that computer readable codes are stored and executed in a distributed fashion.

Further, the program command may include a machine language code generated by a compiler and a high-level language code executable by a device for electronically processing information, for example, a computer through an interpreter and the like.

The hardware device may be configured to operate as one or more software modules in order to perform operations of the present invention, and vice versa.

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above teachings. For example, each component explained in a single form may be provided in a distributed form, and contrarily, each component explained in a distributed form may be provided in a coupled form.

The embodiments of the present invention have been disclosed in the specification and drawings. In the description of the present invention, special terms are used not to limit the present invention and the scope of the present invention as defined in claims, but just to explain the present invention. Therefore, persons skilled in the relevant art can appreciate that many modifications and variations are possible in light of the above teachings. It is therefore intended that the scope of the invention be limited not by this detailed description, but rather by the claims appended hereto.

[Industrial Applicability]

The present invention is applicable to a data processing method and system using auto-thresholding.

**Claims**

1.  A data processing method using auto-thresholding, comprising the steps of:

      receiving, as an input, a plurality of individual numerical values included in a reference data set having two or more clusters through a data processing system;
      calculating a threshold for classifying the clusters the reference data set has through the data processing system, based on the respective numerical values included in the reference data set received; and
      classifying at least one or more analysis subject data sets having a plurality of individual numerical values into different clusters using the threshold through the data processing system.

2.  The data processing method according to claim 1, further comprising the step of calculating a baseline value of the cluster having the smallest average value among the clusters the reference data set has, through the data processing system, based on the individual numerical values included in the reference data set received, the step of classifying at least one or more analysis subject data sets having a plurality of individual numerical values into different clusters using the threshold through the data processing system comprising the steps of:

      calculating the baseline value of the cluster having the smallest average value among the clusters the reference data set has through the data processing system, based on the individual numerical values included in the reference data set received;
      calculating a compensation threshold obtained by compensating for the threshold through the data processing system, based on a difference between the baseline value of the reference data set and the baseline value of the analysis subject data sets; and
      classifying the respective numerical values included in the analysis subject data sets through the data processing system, based on the compensation threshold.

3.  The data processing method according to claim 1, wherein the respective numerical values included in the reference data set and the at least one or more analysis subject data sets are amplitude values of fluorescent signals measured for droplets obtained by adding a fluorescent dye thereto to detect a specific mutation and then performing a polymerase chain reaction (PCR) to gene sequences corresponding to the specific mutation.

4.  The data processing method according to claim 1, wherein the step of calculating a threshold for classifying the clusters the reference data set has through the data processing system, based on the respective numerical values included in the reference data set received comprises the steps of:

      producing histogram data having a plurality of bins with a predetermined bin width using the respective numerical values included in the reference data set through the data processing system;
      performing a noise removing process for allowing the bins having frequencies less than a predetermined noise reference value to have zero frequencies and thus producing histogram data from which noise is removed through the data processing system;
      searching a first target bin existing on the left end of a first cluster in the reference data set through the data processing system, based on the histogram data from which the noise is removed;
      searching a second target bin existing on the right end of a second cluster in the reference data set through the data processing system, based on the histogram data from which the noise is removed; and
      calculating the threshold as any one of the numerical values between the first target bin and the second target bin.

5.  The data processing method according to claim 4, wherein the step of producing histogram data having a plurality of bins with a predetermined bin width using the respective numerical values included in the reference data set through the data processing system comprises the steps of:

      producing an updated data set from which given top-level numerical values and given bottom-level numerical values are removed from the respective numerical values included in the reference data set; and
      producing the histogram data using the respective numerical values included in the updated data set.

**6.** The data processing method according to claim 1, wherein the step of calculating a threshold for classifying the clusters the reference data set has through the data processing system, based on the respective numerical values included in the reference data set received comprises the steps of:

(a) producing histogram data by classifying the range of the numerical values into a plurality of bins having given widths to allow the number of individual data having the respective numerical values of the classified bins to have the frequencies of the respective bins through the data processing system;
(b) performing histogram data equalizing through the data processing system;
(c) performing differencing for the equalized histogram data through the data processing system;
(d) searching a first target bin satisfying a given reference condition and existing on the left end of a first cluster in the reference data set through the data processing system, based on the histogram data with the differencing;
(e) searching a second target bin satisfying the given reference condition and existing on the right end of a second cluster in the reference data set through the data processing system, based on the histogram data with the differencing; and
(f) calculating the threshold as any one of the numerical values between the first target bin and the second target bin through the data processing system.

**7.** The data processing method according to claim 6, further comprising the steps of:

reducing the bin width by a given value through the data processing system if the first target bin or the second target bin satisfying the given reference condition is not searched; and
performing the steps (a) to (e) again using the reduced bin width through the data processing system.

**8.** The data processing method according to claim 1, wherein the step of calculating a threshold for classifying the clusters the reference data set has through the data processing system, based on the respective numerical values included in the reference data set received comprises the steps of:

(a) producing histogram data by classifying the range of the numerical values into a plurality of bins having given widths to allow the number of individual data having the respective numerical values of the classified bins to have the frequencies of the respective bins through the data processing system;
(b) performing histogram data equalizing through the data processing system;
(c) searching a first target bin satisfying a given reference condition and existing on the left end of a first cluster in the reference data set through the data processing system, based on the equalized histogram data; and
(d) searching a second target bin satisfying the given reference condition and existing on the right end of a second cluster in the reference data set through the data processing system, based on the equalized histogram data.

**9.** A computer program installed in the data processing system to execute the data processing method according to any one of claims 1 to 8.

**10.** A computer readable recording medium for recording a computer program for executing the data processing method according to any one of claims 1 to 8.

**11.** A data processing system using auto-thresholding, comprising:

an input module for receiving, as an input, a plurality of individual numerical values included in a reference data set having two or more clusters;
a threshold calculation module for calculating a threshold for classifying the clusters the reference data set has, based on the respective numerical values included in the reference data set received; and
a processing module for classifying at least one or more analysis subject data sets having a plurality of individual numerical values into different clusters using the threshold.

**12.** The data processing system according to claim 11, further comprising a baseline value calculation module for calculating a baseline value of the cluster having the smallest average value among the clusters the reference data set has, based on the individual numerical values included in the reference data set received, the processing module being adapted to divide the at least one or more analysis subject data sets having the plurality of individual numerical values into different clusters using the threshold by calculating the baseline value of the cluster having the smallest average value among the clusters the analysis subject data sets have, based on the individual numerical values included in the reference data set received, calculating a compensation threshold obtained by compensating for the threshold, based on a difference between the baseline value of the reference data set and the baseline value of the analysis subject data sets, and classifying the respective numerical values included in the analysis subject data sets, based on the compensation threshold.

13. The data processing system according to claim 11, wherein the threshold calculation module produces histogram data having a plurality of bins with a predetermined bin width using the respective numerical values included in the reference data set, performs a noise removing process for allowing the bins having frequencies less than a predetermined noise reference value to have zero frequencies to produce histogram data from which noise is removed, searches a first target bin existing on the left end of a first cluster in the reference data set, based on the histogram data from which the noise is removed, searches a second target bin existing on the right end of a second cluster in the reference data set, based on the histogram data from which the noise is removed, and calculates the threshold as any one of the numerical values between the first target bin and the second target bin.

14. The data processing system according to claim 13, wherein the threshold calculation module produces an updated data set from which given top-level numerical values and given bottom-level numerical values are removed from the respective numerical values included in the reference data set and produces the histogram data using the respective numerical values included in the updated data set.

15. The data processing system according to claim 11, wherein the threshold calculation module produces histogram data by classifying the range of the numerical values into a plurality of bins having given widths to allow the number of individual data having the respective numerical values of the classified bins to have the frequencies of the respective bins, performs histogram data equalizing, performs differencing for the equalized histogram data, searches a first target bin satisfying a given reference condition and existing on the left end of a first cluster in the reference data set, based on the histogram data with the differencing, searches a second target bin satisfying the given reference condition and existing on the right end of a second cluster in the reference data set, based on the histogram data with the differencing, and calculates the threshold as any one of the numerical values between the first target bin and the second target bin.

16. The data processing system according to claim 15, wherein the threshold calculation module reduces the bin width by a given value if the first target bin or the second target bin satisfying the given reference condition is not searched, performs the histogram data again using the reduced bin width, and searches the target bin existing on the end of the specific cluster using the histogram data produced again.

17. The data processing system according to claim 11, wherein the threshold calculation module produces histogram data by classifying the range of the numerical values into a plurality of bins having given widths to allow the number of individual data having the respective numerical values of the classified bins to have the frequencies of the respective bins, performs histogram data equalizing, searches a first target bin satisfying a given reference condition and existing on the left end of a first cluster in the reference data set, based on the equalized histogram data, searches a second target bin satisfying the given reference condition and existing on the right end of a second cluster in the reference data set, based on the equalized histogram data, and calculates the threshold as any one of the numerical values between the first target bin and the second target bin.

## FIG. 1A

## FIG. 1B

FIG. 2

# FIG. 3

Receiving individual numerical values included in a
reference data set
S100

Calculating a threshold for classifying the clusters the
reference data set has, based on the individual numerical
values included in the reference data set
S110

With respect to at least one or more analysis subject
data sets
S120

Classifying the individual numerical values included
in the analysis subject data sets using the threshold of
the reference data set
S130

# FIG. 4

S111

| Producing histogram data |

S112

| Producing histogram data from which noise is removed |

S113

| Searching a first target bin existing on a left boundary of a first cluster of the histogram data from which noise is removed |

S114

| Searching a second target bin existing on a right boundary of a second cluster of the histogram data from which noise is removed |

S115

| Calculating a threshold as any one of values between the first target bin and the second target bin |

FIG. 5A

## FIG. 5B

FIG. 5C

Frequencies

Searching
direction(b)

(3-2)

(3-1)

(2-2)

(2-1)

(1-2)

(1-1)

0

T

(3)

(2)

(1)

# FIG. 6A

| Producing updated data set from which given top-level numerical values and given bottom-level numerical values are removed from the respective numerical values included in the reference data set | S1110 |

$\downarrow$

| Producing updated data set from which given top-level numerical values and given bottom-level numerical values are removed from the respective numerical values included in the reference data set | S1120 |

## FIG. 6B

Frequencies

# FIG. 7

O : 1.23425, 2.13425, 4.23252, 3.13141, 1.14452 ...

# FIG. 8

S300

```
┌──────────────────────────────┐
│ Histogram data producing     │
└──────────────────────────────┘
```

S310

```
┌──────────────────────────────┐
│       Equalization           │
└──────────────────────────────┘
```

S320                                                    S340

```
        ◇ Case ◇ ─────────────→ ┌──────────────────┐
                                 │    Searching     │
                                 └──────────────────┘
```

S330

```
┌──────────────────────────────┐
│       Differencing           │
└──────────────────────────────┘
```

FIG. 9

# FIG. 10

S200

Receiving individual numerical values included in a reference data set

S210

Calculating a threshold based on the individual numerical values included in the reference data set

S220

Calculating a baseline value of a cluster having the smallest average value among the clusters the reference data set has

S230

With respect to at least one or more analysis subject data sets

S240

Calculating a baseline value of a cluster having the smallest average value among the clusters the analysis subject data sets have

S250

Calculating compensation threshold obtained by compensating for the threshold by the difference between the baseline value of the reference data set and the baseline value of the analysis subject data sets

S260

Classifying the respective numerical values included in the analysis subject data sets, based on the compensation threshold of the reference data set

# FIG. 11

# FIG. 12

## INTERNATIONAL SEARCH REPORT

| International application No. |
|---|
| **PCT/KR2020/009095** |

### A. CLASSIFICATION OF SUBJECT MATTER

**G16B 50/00**(2019.01)i; **G16B 20/20**(2019.01)i; **G16H 50/70**(2018.01)i

According to International Patent Classification (IPC) or to both national classification and IPC

### B. FIELDS SEARCHED

Minimum documentation searched (classification system followed by classification symbols)

G16B 50/00; C12Q 1/68; G01N 15/14; G01N 33/48; G01N 33/53; G01N 35/00; G01N 37/00; G06F 15/18; G06F 17/00; G06T 7/00; G16B 20/20; G16H 50/70

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 경계 값(threshold), 기저선(baseline), 자동 경계화(auto-thresholding), 데이터 세트(data set), 액적(droplet)

### C. DOCUMENTS CONSIDERED TO BE RELEVANT

| Category* | Citation of document, with indication, where appropriate, of the relevant passages | Relevant to claim No. |
|---|---|---|
| X<br>Y<br>A | KR 10-1782364 B1 (ANI. CO., LTD.) 27 September 2017. See paragraph [0049]; and claim 1. | 1,11<br>3,9-10<br>2,4-8,12-17 |
| Y | JP 2014-507134 A (THE CHINESE UNIVERSITY OF HONG KONG) 27 March 2014. See paragraphs [0011], [0045], [0046] and [0123]-[0128]; and claim 1. | 3,9-10 |
| A | JP 2004-501358 A (BECTON DICKINSON & CO.) 15 January 2004. See claims 1, 2 and 4. | 1-17 |
| A | KR 10-2007-0061347 A (ELECTRONICS AND TELECOMMUNICATIONS RESEARCH INSTITUTE) 13 June 2007. See pages 4-6. | 1-17 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 December 2020** | **07 December 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

## INTERNATIONAL SEARCH REPORT

| | International application No. |
|---|---|
| | **PCT/KR2020/009095** |

**C.    DOCUMENTS CONSIDERED TO BE RELEVANT**

| Category* | Citation of document, with indication, where appropriate, of the relevant passages | Relevant to claim No. |
|---|---|---|
| A | KR 10-2006-0030115 A (A&T CORPORATION et al.) 07 April 2006. See claims 9, 13 and 17. | 1-17 |

Form PCT/ISA/210 (second sheet) (July 2019)

**INTERNATIONAL SEARCH REPORT**
Information on patent family members

International application No.

**PCT/KR2020/009095**

| Patent document cited in search report | | | Publication date (day/month/year) | Patent family member(s) | | | Publication date (day/month/year) |
|---|---|---|---|---|---|---|---|
| KR | 10-1782364 | B1 | 27 September 2017 | None | | | |
| JP | 2014-507134 | A | 27 March 2014 | AU | 2012-204748 | A1 | 02 May 2013 |
| | | | | AU | 2012-204748 | A1 | 12 July 2012 |
| | | | | AU | 2012-204748 | B2 | 24 September 2015 |
| | | | | AU | 2015-227510 | A1 | 08 October 2015 |
| | | | | AU | 2015-227510 | B2 | 20 April 2017 |
| | | | | CA | 2823618 | A1 | 12 July 2012 |
| | | | | CN | 103459614 | A | 18 December 2013 |
| | | | | CN | 103459614 | B | 02 December 2015 |
| | | | | EP | 2661507 | A1 | 13 November 2013 |
| | | | | EP | 2661507 | B1 | 08 April 2020 |
| | | | | EP | 3680909 | A1 | 15 July 2020 |
| | | | | JP | 6105485 | B2 | 05 April 2017 |
| | | | | US | 10152568 | B2 | 11 December 2018 |
| | | | | US | 2014-0019064 | A1 | 16 January 2014 |
| | | | | US | 2019-042693 | A1 | 07 February 2019 |
| | | | | WO | 2012-093331 | A1 | 12 July 2012 |
| JP | 2004-501358 | A | 15 January 2004 | EP | 1295243 | A2 | 26 March 2003 |
| | | | | EP | 2565826 | A1 | 06 March 2013 |
| | | | | EP | 2565826 | B1 | 06 November 2019 |
| | | | | US | 2002-029235 | A1 | 07 March 2002 |
| | | | | US | 2005-042760 | A1 | 24 February 2005 |
| | | | | US | 6809804 | B1 | 26 October 2004 |
| | | | | US | 6944338 | B2 | 13 September 2005 |
| | | | | WO | 01-85914 | A2 | 15 November 2001 |
| | | | | WO | 01-85914 | A3 | 04 April 2002 |
| KR | 10-2007-0061347 | A | 13 June 2007 | EP | 1796009 | A2 | 13 June 2007 |
| | | | | EP | 1796009 | A3 | 22 August 2007 |
| | | | | JP | 2007-157164 | A | 21 June 2007 |
| | | | | KR | 10-0875915 | B1 | 26 December 2008 |
| | | | | US | 2007-0136277 | A1 | 14 June 2007 |
| | | | | US | 7716169 | B2 | 11 May 2010 |
| KR | 10-2006-0030115 | A | 07 April 2006 | CN | 1826529 | A | 30 August 2006 |
| | | | | CN | 1826529 | B | 01 December 2010 |
| | | | | CN | 1826529 | C | 30 August 2006 |
| | | | | EP | 1703283 | A1 | 20 September 2006 |
| | | | | JP | 4150044 | B2 | 17 September 2008 |
| | | | | KR | 10-1106201 | B1 | 20 January 2012 |
| | | | | US | 2006-0190195 | A1 | 24 August 2006 |
| | | | | US | 8676510 | B2 | 18 March 2014 |
| | | | | WO | 2005-008254 | A1 | 27 January 2005 |
| | | | | WO | 2005-008254 | A1 | 31 August 2006 |

Form PCT/ISA/210 (patent family annex) (July 2019)